# EUROPEAN PATENT APPLICATION

(11) **EP 4 481 749 A1**
(43) Date of publication of application: **25.12.2024**
(21) Application number: 23192798.9
(22) Date of filing: 22.08.2023
(51) Int. Cl.: G16H 10/00, G06F 3/01, G06Q 10/0639

(54) **ADJUSTMENT OF WORKPLACE CONDITIONS TO AID USER ATTENTION**

(30) Priority: 20.06.2023 US 202363472365 P
(71) Applicant: Koninklijke Philips N.V., 5656 AG Eindhoven (NL)
(72) Inventor: WIJN, Victor, 5656 AG Eindhoven (NL); KUHLMANN, Daan, 5656 AG Eindhoven (NL); BUIL, Vincentius Paulus, 5656 AG Eindhoven (NL); TUINHOUT, Jelle Jeroen, 5656 AG Eindhoven (NL); SEVENSTER, Merlijn, 5656 AG Eindhoven (NL); VOSBERGEN, Sandra, 5656 AG Eindhoven (NL); HILLEN, Marleen, 5656 AG Eindhoven (NL); KNOESTER, Jaap, 5656 AG Eindhoven (NL); NGUYEN, Duy Duc, 5656 AG Eindhoven (NL); LAMB, Hildo, 5656 AG Eindhoven (NL)
(74) Representative: Philips Intellectual Property & Standards

(57) **Abstract**

Generating attention data of a user, wherein the attention data comprises at least user sensor data and a current case status, wherein the current case status comprises one of a case complexity, a case urgency, or a progression of image review, determining the occurrence of an interruption or a change in the attention data and performing an adjustment of one or more workspace items.

## Description

### FIELD OF THE INVENTION

The present invention relates to the field of medical imaging arts, radiology arts, radiology reading arts, radiology workstation arts, workstation user interfacing arts, medical coding arts, and related arts.

### BACKGROUND OF THE INVENTION

Use of digital technologies in radiology has caused a substantial increase in the quantity of clinical image and non-image information used by healthcare professionals for diagnosis. Despite this growth, clinical assessments are still performed in the traditional fashion. This incongruence has caused large increases in workloads and data overload for healthcare professionals.

Efficiency is a primary goal of modern healthcare systems (i.e., doing more with less). Gains in efficiency are often at the expense of increasing workloads on radiologists and technicians. Increased workloads and stress on medical professionals represent a counterweight to nominal efficiency gains and may drive staff burnout and incorrect image analysis. In other words, improving the experience of medical professionals is a key component of an efficient healthcare system.

Radiologist time/availability is a scarce resource. Various distractions vie for that time (e.g., emails, calls, background chatter, etc.). Technicians and requesting physicians frequently make inquiries of radiologists. These inquiries may derail the focus and workflow of a radiologist. Frequent task switching, review inefficiency, and user frustration are all components of a negative work environment of a radiologist.

Moreover, it is more impactful and less desirable to interrupt a radiologist reviewing a complicated patient case compared to a relatively less complicated case, because significantly more time is required in a complicated case for the radiologist to return to the place in the radiology reading workflow where he left off prior to the interruption and regain sufficient understanding of the case at that place in the workflow. This may be understood as an increased "cost of distraction". These higher costs of distraction may lead to additional stress on radiologists, leading to potential image analysis errors due to distraction.

In the light of the current dynamics in healthcare worldwide (e.g., declining reimbursements, value-driven healthcare policies, shortage of medical staff worldwide and high sick leave rates because of work stress), improvements to the distraction environment of radiologists are urgently needed.

### SUMMARY OF THE INVENTION

According to examples in accordance with an aspect of the invention, there is provided a method, comprising generating attention data of a user, wherein the attention data comprises at least user sensor data and a current case status. wherein the current case status comprises one of a case complexity, a case urgency, or a progression of image review. The method further comprises determining the occurrence of an interruption or a change in the attention data; and performing an adjustment of one or more workspace items.

In some embodiments, the user sensor data comprises one of user eye-gaze behavior, user posture, user heart-rate variability, user skin conductance, user pupil dilation, or detected distractions nearby a user.

In some embodiments, the adjustment is based on a focus preference.

In some embodiments, performance of the adjustment further comprises adjusting at least one of a noise cancellation system, a lighting control system, an ergonomics system, a scent system, or a sound system.

In some embodiments, determining the occurrence of an interruption further comprises a user state, the user state comprising at least one of attentiveness, fatigue, or stress.

In some embodiments, the user state is a quantized value corresponding to a predefined metric.

In some embodiments, the performance of the adjustment is based on the quantized value.

According to further examples in accordance with an aspect of the invention, there is provided a method comprising receiving an inquiry related to a current case; determining an urgency of the inquiry; determining a user availability score. The method further comprises determining whether to interrupt a user based on the urgency and user availability score.

In some embodiments, the method further comprising (i) determining a caller intent related to the inquiry; (ii) determining an urgency score based on the urgency of the inquiry and (iii) organizing the inquiry into a prioritized list, based on the urgency score; and when a combined user availability score and urgency score satisfies a predefined threshold value, notifying the user of the inquiry.

In some embodiments, the method further comprising determining whether the inquiry is a call from a live caller; and when the inquiry is a call from a live caller, generating a case ticket from a phone-based dialogue interface provided to the caller.

In some embodiments, the determining a caller intent includes determining an urgency score of the inquiry, and determining a user availability score are based on at least one of machine learning or natural language processing.

In some embodiments, the urgency score of the inquiry comprises at least one of an estimated time needed for the user to provide an answer, a question complexity, a question urgency, and a question type.

In some embodiments, the user availability score is based on at least one of an agenda of the user, a user emotional state, a user location, user movement tracking, user activity, or user stress levels.

In some embodiments, the inquiry is received from a disturber and directed to a receiver and wherein the user availability score is determined separately for the disturber and receiver, wherein the urgency is a time interval to complete the inquiry, and wherein the determining whether to interrupt the user is based on the user availability score for both the disturber and the receiver during the time interval.

According to further examples in accordance with an aspect of the invention, there is provided a method comprising generating first attention data of a user, wherein the first attention data comprises at least user sensor data and a current case status; determining the occurrence of an interruption or a change in the first attention data. The method further comprising performing a first adjustment of one or more workspace items; generating second attention data of the user, wherein the second attention data comprises at least user sensor data and a current case status. The method further comparing the first and second attention data; and performing a second adjustment of the one or more workspace items, based on the comparison.

In some embodiments, the user sensor data comprises one of user eye-gaze behavior, user posture, user heart-rate variability, user skin conductance, user pupil dilation, or detected distractions nearby a user.

In some embodiments, first and second adjustment are based on a focus preference.

In some embodiments, performance of the first and second adjustment further comprises adjusting at least one of a noise cancellation system, a lighting control system, an ergonomics system, a scent system, or a sound system.

In some embodiments, determining the occurrence of an interruption further comprises a user state, the user state comprising at least one of attentiveness, fatigue, or stress.

In some embodiments, the user state is a quantized value corresponding to a predefined metric; and the performance of the first adjustment is based on the quantized value.

According to further examples in accordance with an aspect of the invention there is provided a system comprising a processor, wherein the processor is configured to generate attention data of a user, wherein the attention data comprises at least user sensor data and a current case status. wherein the current case status comprises one of a case complexity, a case urgency, or a progression of image review. The processor being further configured to determine the occurrence of an interruption or a change in the attention data; and performing an adjustment of one or more workspace items.

According to further examples in accordance with an aspect of the invention there is provided a system comprising an interface configured to receive an inquiry related to a current case. The system further comprising a processor, the processor being configured to determine an urgency of the inquiry and determine a user availability score. The processor being further configured to interrupt a user based on the urgency and user availability score.

According to further examples in accordance with an aspect of the invention there is provided a system comprising a processor, wherein the processor is configured to comprising generating first attention data of a user, wherein the first attention data comprises at least user sensor data and a current case status; determining the occurrence of an interruption or a change in the first attention data. The method further comprising performing a first adjustment of one or more workspace items; generating second attention data of the user, wherein the second attention data comprises at least user sensor data and a current case status. The method further comparing the first and second attention data; and performing a second adjustment of the one or more workspace items, based on the comparison.

According to additional examples in accordance with another aspect of the invention there is provided a computer program product comprising computer program code means which, when executed on a computing device having a processing system, cause the processing system to perform all of the steps of the method for operating a personal care device comprising a movable component and an actuator according to an embodiment of the invention.

These and other aspects of the invention will be apparent from and elucidated with reference to the embodiments described hereinafter.

### BRIEF DESCRIPTION OF THE DRAWINGS

Fig. 1 shows a schematic drawing of a system according to an exemplary embodiment.
Fig. 2 shows a schematic drawing of a wearable, according to various exemplary embodiments.
Fig. 3 shows a flow diagram for adaptive workspace focus enhancement, according to various exemplary embodiments.
Fig. 4 shows a flow diagram for holistic notification management, according to various exemplary embodiments.
Fig. 5 shows a flow diagram 500 for interruption management based on urgency, according to various exemplary embodiments.
Fig. 6 shows an exemplary graph plotting the interruptability curves of two stakeholders according to various exemplary embodiments.

### DETAILED DESCRIPTION OF THE EMBODIMENTS

The exemplary embodiments may be further understood with reference to the following description and the related appended drawings, wherein like elements are provided with the same reference numerals. The exemplary embodiments relate to use of an adaptive focus environment to assist a user to maintain focus on their work. The exemplary embodiments further pertain to contextually aware interruption management of a user based on a user context (e.g., a user that is moving about a location such as a hospital, a user that is currently focused on a task, etc.)

The exemplary embodiments are described with reference to a radiologist. However, it should be understood that the "user" of the exemplary systems may be any person engaged in an activity where interruptions may disrupt the workflow of the activity. Some other examples of a user may include but are not limited to a technician or technologist, a clinician such as a doctor, nurse, or other type of medical practitioner.

As discussed above, a user (e.g., a radiologist) is faced with numerous distractions throughout the workday, reducing their efficiency. Existing radiology platforms may enable communication regarding case tickets and questions. A radiologist can follow a worklist that is updated and prioritized automatically based on urgency indicated by a technician. A radiologist can further note outcomes immediately via a chat function directly with the appropriate stakeholder assigned to that specific case ticket.

A typical workday of a radiologist will feature several interruptions (e.g., background noise and visual distraction). These disturbances may occur at inopportune moments, which may result in a disruption of a user workflow, as well as increases in cognitive load, frustration, and stress. When a radiologist is deeply engaged with reviewing urgent and/or complex cases, it can be assumed that disturbances may lead to a higher "cost of distraction". These distractions result in inefficient image review cycles and a significant decrease in staff experience for the radiologists as well as the technologists. It is therefore in the interest of radiologists, healthcare systems, and patients that these distractions are minimized. A comfortable working environment that adaptively enhances the focus of a user is a potential solution to this wide-ranging issue.

Analysis of various physiological data of a user (e.g., a technician or technologist, a clinician such as a doctor, radiologist, nurse, or other type of medical practitioner) may be used to generate an adaptive (i.e., "smart") focus environment. This adaptive focus environment may be modified to adjust the working environment of a user to increase focus.

In a first aspect of the exemplary embodiments, a method of data collection to adapt a working environment to enhance the focus of a user is disclosed herein. Data to be collected may include personal, interactional, and contextual information around the radiologist and/or the workstation. An adaptive workspace may be understood to represent a working environment that has reactively and/or proactively minimized potential further distractions through hardware and software solutions. In a general sense, as distraction increases, so too do the measures of an adaptive workspace.

At a high level of abstraction, adaptive workspaces may be an extension of the existing platforms to optimize user focus in critical moments. An adaptive workspace need not only operate on the sensed parameters of the user, but also based on current case information. Together, this information may be used for a holistic distraction mitigation system that is adaptive to a given environment, case, and radiologist.

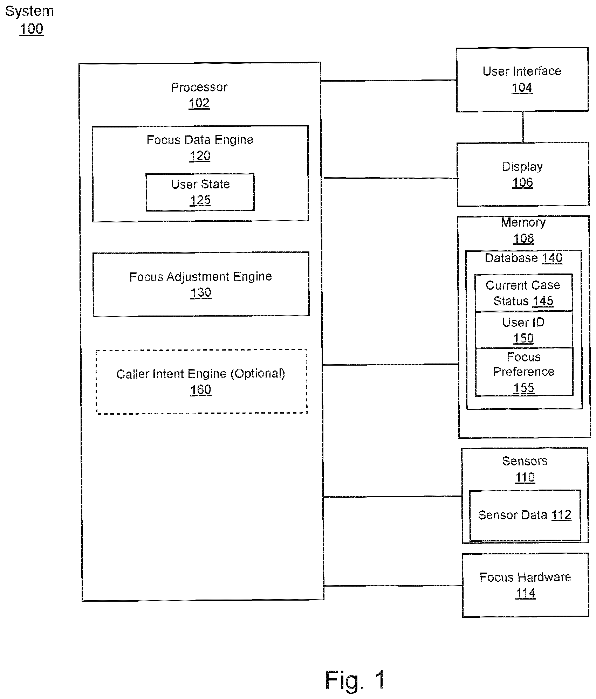

Fig. 1 shows a schematic drawing of a system 100 according to various exemplary embodiments. The system 100 comprises a processor 102, a user interface 104, a display 106, and a memory 108. The memory 108 includes a database 140, which stores the medical records of patients. The medical records may include clinical data from a variety of sources such as, for example, medical images (e.g., MRI, CT, CR ultrasound), problem lists, lab values, medication lists, and documents including admissions and discharge notes and pathology, radiology, and operation reports.

In some embodiments, the system 100 may comprise a radiology workstation operated by a radiologist. The subcomponents of system 100 may not all be located in the same location (for example, database 140 may not need to be physically present with a user but may be located off-site on a server). Other subcomponents of system 100 such as sensors 110 may be distributed throughout a workspace (e.g., cameras located overhead of a user).

In some embodiments, the system 100 may, for example, constitute or may comprise a Picture Archive and Communication System ("PACS") which according to Wikipedia, "is a medical imaging technology which provides economical storage and convenient access to images from multiple modalities (source machine types)." A PACS can be designed in many different ways. An exemplary PACS technology infrastructure may include imaging device interfaces, storage devices, host computers, communication networks, and display systems often integrated by a flexible software package for supporting a radiologist in reading a patient case through a diagnostic workflow. Common hardware components may include for example, patient data servers, data/modality interfaces, PACS controllers with database and archive, and display workstations connected by communication networks for handling the efficient data/image flow. However, the system 100 may also be a standalone PACS workstation. The system 100 may also be a teleradiology system or workstation (including for example a PACS) operating remotely from a hospital and using the cloud for communication with hospital systems such as imaging modalities.

In other embodiments, the system 100 may be an imaging system console (e.g., MR or CT console) where a technician or technologist is conducting one or more scans of a subject. System 100 may also be a standalone workstation.

The database 140 may contain a current case status 145 for a case under review or to be reviewed by a radiologist. A case may also be referred to as an image study. The current case status 145 may provide information such as (1) the general complexity of the case (which may be determined by analyzing the content of the case itself), (2) case urgency (analyzed by the context, e.g., a patient waiting on an examination table) and/or time criterion, and (3) the progression of the image review (the extent to which the case has already been reviewed by the radiologist). Case progression provides information on the completeness of a radiologist workflow or review, and the speed at which the review is being performed. The database 140 may also contain stored information associated with user credentials which can be used to define different operational aspects of the system 100 specific to different users as will be described in more detail below.

The system 100 includes sensors 110 which acquire sensor data 112 about a user. The sensors 110 may include a variety of hardware and software that can generate sensor data 112 such as for example, but not limited to, microphone, light sensor, accelerometer, gyroscope, camera, heart rate sensor (determined by a device worn by a user, such as the wearable 200), and skin conductance sensor (determined by a device worn by a user, such as the wearable 200). It is noteworthy that while the wearable 200 may include multiple sensors such as a heart rate sensor and skin conductance sensor, these sensors may also exist or be independently used in different or separate wearables or devices. The sensor data 112 includes for example one or a combination of eye-gaze behavior, posture, heart-rate variability, skin conductance, and distraction sensing of a user.

The processor 102 executes a focus data engine 120 for performing operations such as processing sensor data 112 and determining and/or quantifying a user state 125 to be used by focus adjustment engine 130 for controlling and/or adjusting the focus hardware 114 and/or controlling and/or adjusting software functions to improve the user focus. A user state 125 is for example attentiveness, fatigue, and/or stress of a user. The focus hardware 114 is for example one or more device(s) that are controlled by the focus adjustment engine 130 to perform one or more actions (or controls) or adjustments to enhance the focus of a user in response to the determined or quantified user state 125. A non-exhaustive list of examples of focus hardware 114 is provided below. The focus data engine 120 may also incorporate the user ID 150 and the focus preferences 155 into its determination of the user state 125. Determination of a user state 125 will be discussed in further detail below.

The focus hardware 114 includes for example, but is not limited to, noise cancellation hardware, speakers, adaptive lighting, motors capable of adjusting the ergonomics of a workstation (e.g., a chair, display 106, or a desk), a scent diffuser/vaporizer, and motors capable of moving a distraction barrier (for example, raising one or more panels around display 106). The focus adjustment engine 130 may produce outputs that adjust the focus hardware 114 such as adjusting noise cancellation parameters (e.g., scale and intensity), soundscapes (e.g., alpha waves, rain sounds, etc.), lighting of a room (e.g., ambiance, pulsing, colour, brightness), movable panelling around the user, concentration-promoting scents (and their respective type and intensity), and chair/desk/display position.

The focus hardware 114 may be controlled and/or adjusted to achieve an enhanced focus state semi-automatically (with user input through user interface 104) or automatically without any user input. For example, a user may be prompted to input any of the various settings of the focus hardware 114 described above, e.g., lighting level, chair position, etc. based on, for example, the sensor data 112 or to confirm the control and/or adjustments to be made by the focus adjustment engine 130. On the other hand, if the focus hardware 114 is controlled and/or adjusted automatically, these controls/adjustments to the focus hardware 114 may be performed without user input or confirmation.

As mentioned above, focus adjustment engine 130 may also control and/or adjust software such as, for example, software parameters on a user device, such as by enabling a do-not-disturb feature. Such a do-not-disturb feature may prevent notices from appearing on the user's display and distracting the user during crucial medical review workflow. Focus adjustment engine 130 may adjust and/or control software in addition to or in place of focus hardware 114.

The database 140 may associate a given user ID 150 with a predefined focus preference 155. The focus preference 155 may define the limits of control/adjustment that the system 100 may take to regain or maintain the focus of a user. For example, a user with a more "aggressive" focus preference 155 may experience more forceful control/adjustment by the system 100 as compared to a less aggressive preference 155. An example of aggressive behavior by system 100 may be adjusting lighting more quickly compared to a lighting adjustment operating with a less aggressive user preference 155. A user may adjust their focus preference 155. It should be understood that the focus preference 155 need not be defined by an operating user; it could also be defined by an administrator (e.g., aggressive for all radiologists) for a radiology department, medical center, hospital, or similar.

The processor 102 may optionally execute a caller intent engine 160. Caller intent engine 160 will be described in further detail with respect to the second aspect of the exemplary embodiments, though it should be noted that the caller intent engine 160 may be included with the first aspect of the exemplary embodiments without altering the scope of the disclosure herein.

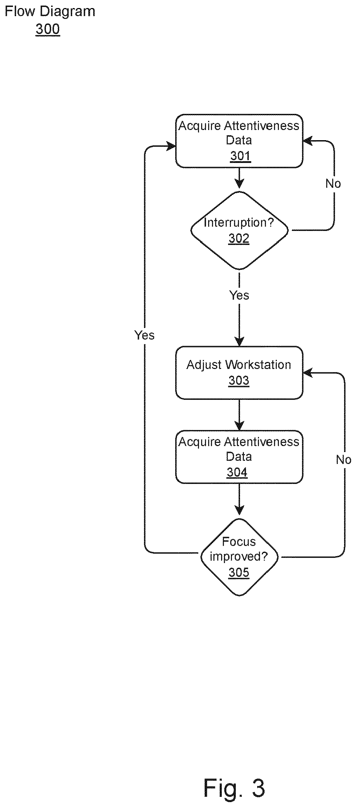

In a different embodiment, the focus data engine 120 may perform analysis on sensor data 112 through AI models to generate a user state 125. The user state 125, as determined through AI models in this embodiment, may be used by the focus adjustment engine 130 to control and/or adjust the focus hardware 114. The focus data engine 120 may continually monitor sensor data 112 and the user state 125 to determine if the focus adjustment engine 130 should continue adjusting focus hardware 114 until a user is focused again. In other words, there is a feedback loop between the focus hardware 114, the focus data engine 120, the user state 125, the focus adjustment engine 130, and the sensor data 112. The feedback loop may be adjusted based on user preference, if desired. Further description of the feedback loop may be found with respect to Fig. 3.

Distraction (e.g., attentiveness) may be measured by the focus data engine 120 via the sensors 110 and the sensor data 112. Distraction may be determined through measurements of eye-gaze behavior, pupil dilation, body posture, and distraction sensing. Distraction determinations may indicate whether a user is looking at the display 106. For example, pupil diameter may indicate how engaged a user is with what they are looking at or focused on.

The distraction determination may be further derived from measurements of a user interactional state (e.g., key presses, mouse behavior, interface selections, and states in an application running on system 100), or measuring a contextual state (e.g., other people present in addition to the user, conversational analysis, etc.).

The focus data engine 120 may determine fatigue through similar measurements as discussed above with respect to distraction. Fatigue analysis may instead focus on eye-openness and the frequency at which a user is distracted or looking away from the display 106.

The focus data engine 120 may determine a stress level of a user by combining heart rate variability and skin conductance (or galvanic skin response (GSR)) information from the sensor data 112.

It should be understood that distraction, fatigue, and stress may be three different user states 125. It is possible that these user states 125 could be combined to arrive at a holistic determination of a user state, or individually used to drive the focus adjustment engine 130. It should be further understood by one of skill in the art that sensor data 112 could be used to determine numerous additional user states depending on operator need. In some examples, the system 100 has only one user state at a time. For example, if the system 100 is tracking distraction and stress and determines that the user is distracted and stressed, that would be a single user state considered as "distracted+stressed". The focus adjustment engine 130 would respond accordingly to the defined behavior for distracted+stressed, which may be different than the "distracted" or "stressed" user states.

It may be advantageous to assign different levels of intervention (e.g., control and/or adjustment) to the focus adjustment engine 130 and focus hardware 114 based on the user state 125. The focus data engine 120 may assign a numerical score based on sensor data 112 as part of determining or quantifying the user state 125. For example, based on eye measurements (and/or other measurements), the system 100 may determine that the user state 125 is in a first quartile for distraction. The user state 125 for first quartile distraction may be associated with control/adjustment of the focus hardware 114 by the focus adjustment engine 130. In this way, an operator could define different control/adjustment based on a level of distraction of the user. Typically, a more distracted a user may have "harsher" measures activated, e.g., the more distracted a user the brighter the lights are adjusted. This may include predefined mappings or associations between different levels or degrees of distraction and control/adjustment actions. A simple example of this may be a chair adjustment for a user glancing at their phone (a first quartile for distraction) as compared to bright light flashing in tandem with ergonomic adjustments for a user that is asleep (e.g., the fourth quartile).

One of skill in the art will recognize that reference to quartiles is only exemplary. One of skill in the art will recognize that any rubric for division of a user state into subcategories may be implemented, and that a system may use the focus data engine 120 to determine a user state based on more than one variable (e.g., monitoring for distraction, fatigue, and/or stress simultaneously). It should be apparent that an operator can define different metrics for different quartiles, and that phone use and sleep are only examples. In this manner, it is possible for the focus hardware 114 to be adjusted by the focus adjustment engine 130 in an incremental (i.e., quantized) and adaptive manner to a user or user behavior.

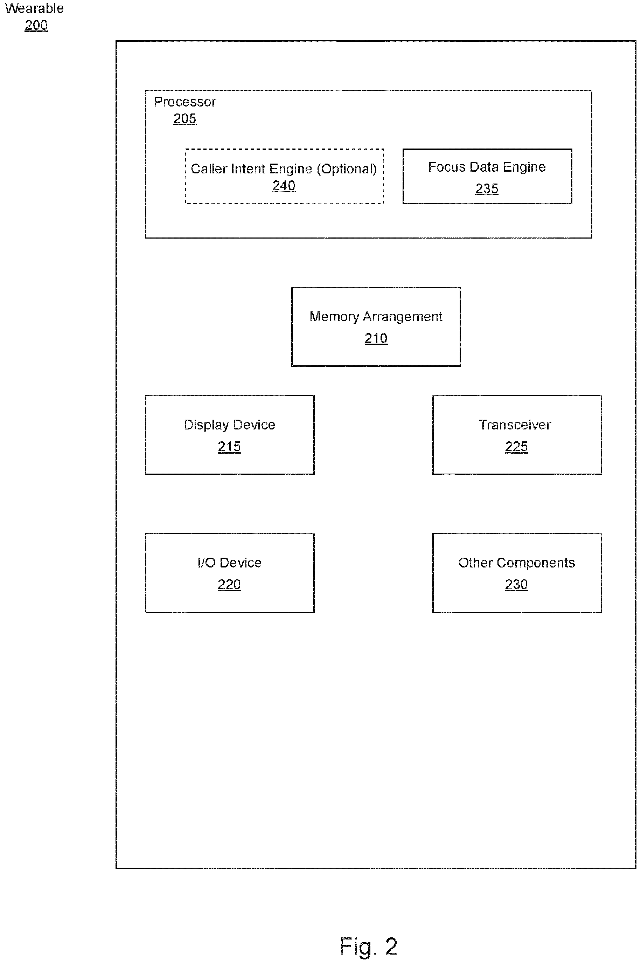

Fig. 2 shows a schematic drawing of a wearable 200, according to various exemplary embodiments. The wearable 200 may represent any electronic device and may include a processor 205, a memory arrangement 210, a display device 215, an input/output (I/O) device 220, a transceiver 225, and other components 230. The other components 230 may include, for example, an audio input device, an audio output device, a battery that provides a limited power supply, a data acquisition device, ports to electrically connect the wearable 200 to other electronic devices, sensors to detect conditions of the wearable 200 (such as an accelerometer, gyroscope and barometer), a heart rate sensor, a skin conductance sensor, etc.

It should be understood that the wearable 200 may be reduced in component complexity depending on the specific implementation of system 100. In some cases, all components of wearable 200 may be present (e.g., a smartwatch, a smartphone, etc.). In other cases, wearable 200 may be as simple as a Radio Frequency Identification (RFID) tag, a Bluetooth Low-Energy (BTLE) tag, etc. While the term "wearable" is used in the present disclosure, the wearable 200 need not be physically worn on a user. For example, wearable 200 could even be a laptop computer that is carried by a healthcare professional or sits within a certain distance of the professional. In summary, wearable 200 is described in its maximally complex state, but may be reduced in complexity depending on operator implementation and needs.

The processor 205 may be configured to execute a plurality of engines for the wearable 200. For example, the engines may include a focus data engine 235 for performing operations such as processing sensor data 112 for the focus adjustment engine 130.

The processor 205 may optionally execute a caller intent engine 240. Caller intent engine 240 will be described in further detail with respect to the second aspect of the exemplary embodiments, though it should be noted that the caller intent engine 240 may be included with the first aspect of the exemplary embodiments without altering the scope of the disclosure herein.

The above referenced engine being an application (e.g., a program) executed by the processor 205 is only exemplary. The functionality associated with the engines may also be represented as a separate incorporated component of the wearable 200 or may be a modular component coupled to the wearable 200, e.g., an integrated circuit with or without firmware. For example, the integrated circuit may include input circuitry to receive signals and processing circuitry to process the signals and other information. The engines may also be embodied as one application or separate applications. In addition, in some wearables, the functionality described for the processor 205 is split among two or more processors such as a baseband processor and an applications processor. The exemplary embodiments may be implemented in any of these or other configurations of a wearable.

The memory arrangement 210 may be a hardware component configured to store data related to operations performed by the wearable 200. The display device 215 may be a hardware component configured to show data to a user while the I/O device 220 may be a hardware component that enables the user to enter inputs. The display device 215 and the I/O device 220 may be separate components or integrated together such as a touchscreen. The transceiver 225 may be a hardware component configured to establish a connection with a cellular network, a wireless local area (WLAN) network, a global navigation satellite system (GNSS) signal, a near-field communication (NFC) connection, and/or a Bluetooth connection. Accordingly, the transceiver 225 may operate on a variety of different frequencies or channels (e.g., set of consecutive frequencies). The transceiver 225 may communicate with system 100.

Fig. 3 shows a flow diagram 300 for adaptive workspace focus enhancement, according to various exemplary embodiments. In 301, a system (e.g., system 100) acquires attentiveness data 304 (e.g., sensor data 112, user state 125, current case status 145, and focus preference 155) continually as a user works. If there is no interruption 302, the system 100 continues to acquire attentiveness data 304. The acquired attentiveness data 304 may be used to generate a user state 125 (e.g., attentive) in the background. The system 100 at this time knows that the user is attentive and continues to monitor the sensor data in the background.

However, in 302, if the system 110 determines that incoming sensor data indicates an interruption (e.g., user looking at phone, nodding off, making little progress through a current case status 145, etc.), the system 110 proceeds to 303 as described below. The determination of an interruption may be understood to be the focus data engine 120 determining a distracted user state 125.

If there is an interruption, the system 100 proceeds to 303. In 303, the system 100 adjusts the workstation of the user. This may be understood as the focus adjustment engine 130 adjusting the focus hardware 114 (e.g., changing the lighting, adjusting ergonomics, activating a do not disturb mode, emitting odors, etc.). The adjustment to the workstation 303 may be based on a quantized rubric, such that different levels of distraction (as determined by the user state 125) cause different workstation adjustments. The quantized rubric may incorporate modifiers based on the current case status 145. As an example, a more complex/less progressed/more urgent case may modify the determined user state 125.

Several examples are illustrative. In a first example, a user that is determined to be focused may still have the workstation adjusted (perhaps being placed in a second quartile for distraction) because the user is working on a complex case. The system 100 may enable focus hardware 114 such as noise cancellation, sound scaping (e.g., rain sounds), and light levels may be adjusted.

In a second example, a user may be determined to be distracted and fatigued, (based on heart rate and eye tracking data). The system 100 may adjust focus hardware 114 such as noise cancellation, soundscaping (e.g., rain sounds), and light levels may be adjusted in different amounts than in the first example.

The pertinent point of the above examples is that an operator may associate user states 125 with different adjustments of focus hardware 114. In some examples, the association between focus hardware 114 and the user state 125 may be defined according to operator preference. An operator may define their own rubric (quartiles, quintiles, binary, etc.) for defining levels of a user state. An operator may create any association between sensor data 112 and a determined user state 125. An operator may select any modifier of their choosing for how a current case status may affect the focus adjustment engine 130. In other examples, the association between focus hardware 114 and the user state 125 may include default settings, e.g., when a particular user state 125 exists, control/adjust the focus hardware 114 in the same manner for all users.

It is possible that adjustment of the workstation 303 may be modified by a manual override of the user. For example, the lights may be brightened, and a user may then dim the lights. The user could also perform a manual control/adjustment on their workstation through software or hardware to modify the adjusted workstation. The manual override may also include a user completely disabling the method of flow diagram 300 (e.g., disabling the attention tracking and adjustment features of the exemplary embodiments).

Following the adjustment 303, the system 100 acquires more attentiveness data 304. The attentiveness data 304 again includes sensor data 112, the current case status 145, and user input and preferences. An example of user input/preference could be a manual override of adjustment 303 by the user. The operation 304 is part of the feedback loop of system 100. If the acquired attentiveness data 304 indicates that the adjustment 303 did not have enough of an effect on the attentiveness of the user, the system 100 may further adjust the workstation 303 (e.g., further brightening a room). If the acquired attentiveness data 304 does indicate that the user is now attentive, the system 100 returns to 301 and reenters a background monitoring state.

The determination of a user state 125 may be useful information for coworkers. For example, a user state 125 may be associated with an "interruption index". For example, a user with a distracted user state in a first quartile may be associated with an interruptible state. This interruptible state may be transmitted by various means such as internal communication services, external communication services, or physical changes to a user workspace (for example, turning on a green light outside the office of a radiologist).

The user emotional state may also be determined by the focus data engine (120). The user emotional state may also be associated with the interruption index, such that a "sad" user may receive an interruptible index associated with do-not-disturb features and adjustments to focus hardware 114. Finally, the interruption index may be associated with a user cognitive state, as determined by the focus data engine 120. If a user is determined to have a high cognitive load, the system may engage do-not-disturb features and adjustments to focus hardware 114.

While the exemplary embodiments are described with respect to a radiologist, it should be apparent that the above-described exemplary embodiments may be applicable to various other fields of use.

In a second aspect of the exemplary embodiments, holistic interruption management techniques and methods for users are disclosed herein. The second aspect of the exemplary embodiments may be understood to complement the first aspect of the exemplary embodiments, such that the first and second aspects form a comprehensive mesh of interruption and focus management.

The second aspect may also include manners for the 'disturber' (e.g., referring physician, nurse, technician, colleague radiologist, etc.) to indicate how urgent the message is to the 'receiver' (e.g., radiologist), e.g., from an emergency (e.g., needs to be dealt with immediately) to anywhere between now and some later time (e.g., 30 minutes, 60 minutes, etc.). In some exemplary embodiments according to the second aspect, an intelligent system (e.g., distraction management system) can determine an ideal time (within the time window) for the radiologist to be disturbed.

There are scenarios where a user may not be at their workstation (e.g., system 100). It follows that during such times, it may be more desirable to contact the user than times when the user is actively working at their workstation. Movement information may be provided by a variety of systems, such as the wearable 200. Additional sensitivity may be added to the proposed system by way of analysis of caller intent. It should be noted that "caller" may refer to both voice and typed communications to a user.

Analysis of caller intent may enhance a distraction management system. Processor 102 may optionally execute a caller intent engine 160. Caller intent engine 160 may determine several useful metrics such as an estimated time needed for a user to provide an answer, question complexity, urgency, and question type to be asked of a user. This may be performed by machine learning (ML) techniques and/or natural language processing (NLP) of a voice inquiry of a user. This information may organize the inbox of a user in such a way that is prioritized based on the aforementioned metrics in a way that maximizes productivity. The optional caller intent engine 240 of wearable 200 performs operations substantially similar to caller intent engine 160.

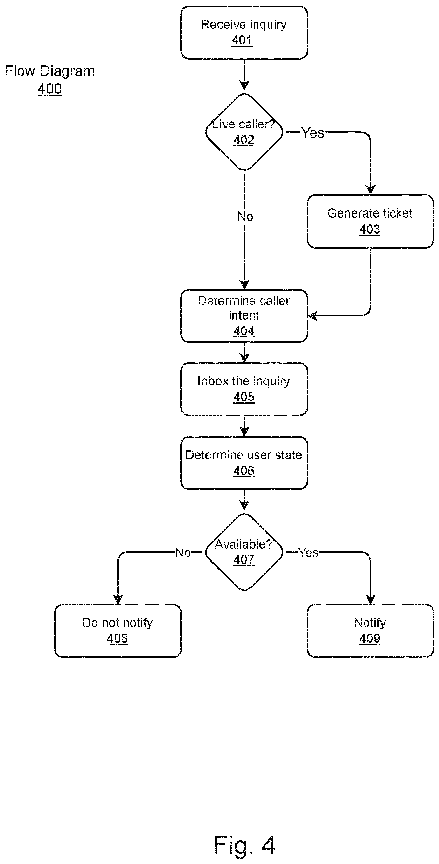

Fig. 4 shows a flow diagram 400 for holistic notification management, according to various exemplary embodiments. Flow diagram 400 may be applicable to scenarios in which a user is utilizing a wearable (e.g., wearable 200) as part of a holistic notification management scheme. Reference to the wearable 200 performing analysis operations are made herein, but is also possible that the system 100 may perform such operations and communicate the results back to the wearable 200 (e.g., via the caller intent engine 160). It is also possible the operations of flow diagram are performed entirely off-site, for example, at a server located in an entirely different location to a user. Some operations of the flow diagram 400 may use hardware that may not be part of the wearable 200, such as cameras for emotional state tracking. In such a case, the focus hardware 114 may be understood to operate in place of the hardware not present on the wearable 200.

In 401, a user receives an inquiry. One of skill in the art will recognize that doctors, technicians, and nurses frequently make information requests of radiologists. The inquiry may arrive as a notification at the wearable 200, or it may arrive at another device such as a laptop or smartphone of a user.

In 402, the wearable 200 determines if the inquiry 401 is a live caller. If the inquiry 401 is not a live caller, the wearable 200 proceeds to 404. If the inquiry 401 is a live caller, the wearable 200 proceeds to 403. In 403, the wearable 200 generates a ticket by routing the caller through a self-help system. The self-help system leads the caller through a series of questions that allows the wearable 200 to generate a ticket identifying the caller. It is also possible the self-help system may have an emergency override feature. Such a feature would enable a caller to bypass any screening or notification management systems to reach the user. In such a case, the self-help system would immediately notify the user in 409.

In 404, the caller intent engine 204 processes the received inquiry 401 to determine the purpose of the call. While the term call is used here, the inquiry 401 need not be a voice call or voicemail but could instead be a text-based message through any of a variety of communications platforms.

In 404, the wearable 200 now has either a generated ticket or a non-live caller inquiry (e.g., email, text message, etc.) The caller intent engine 204 determines an estimated time needed for the user to provide an answer, question complexity, question urgency, and/or question type. The caller intent engine may perform the analysis via, for example, machine learning (ML), natural language processing (NLP), etc.

In 405, the caller intent engine 204 processes the inquiry 401 based on the caller intent 404. The caller intent engine 204 may organize an inquiry based on the estimated time needed for the user to provide an answer, question complexity, question urgency, and/or question type. Such prioritization is customizable based on operator needs. Generally, more complex and time-consuming inquiries will be prioritized in an inbox compared to less complex and time-consuming inquiries. The caller intent engine may assign an "urgency score" to the inquiry 401 based on the aforementioned inquiry data (estimated time needed for the user to provide an answer, question complexity, question urgency, and/or question type). The urgency score may be used with an availability score to determine whether a user should be notified.

In 406, a user state is determined. The user state 406 may be understood to comprise an "availability score". The availability score may be based on an agenda/calendar of the user, a user emotional state, a user location, user movement tracking, and/or user activity sensing and stress levels. These parameters may be used to determine the user availability score. It is possible that this operation may be performed by focus data engine 235, and/or focus data engine 120. User location may be determined by a variety of means, such as GNSS, NFC, Bluetooth (e.g., Bluetooth low-energy beacons), camera-based facial analysis software tracking a user location throughout a building, etc.

An operator and/or user may assign different values to the parameters to generate a holistic notification management system. Examples of parameters that may be tracked are: the user agenda (including previous and upcoming tasks), the user current emotional state (via a facial camera and/or a heartrate tracker as part of other components 230) the user current location and/or velocity (via any suitable location tracking means such as GNSS and/or accelerometer), presence and movement tracking (by camera and/or accelerometer/gyroscope), activity sensing, (via accelerometer data combined with a user agenda), and/or the user stress levels (via heart-rate monitoring or skin conductance).

These parameters may be utilized by, for example, an AI algorithm to determine a user availability score. It is also possible that location data may be used to determine whether the user is going to a location associated with a high stress environment (e.g., an exam room vs. a lunchroom). Such information may also influence the availability score. An emotional state may also influence the availability score, e.g., not notifying a stressed user. Again, an operator is free to define the relationship between agenda, emotional state, location, stress, and causes of movement with the availability score. Typically, an occupied agenda, location in a defined "important" area (such as an exam room), a stressed emotional state, and work-related causes of movement (as opposed to getting a coffee) will be associated with higher availability scores.

It is possible that the wearable 200 and/or the system 100 may track availability scores over time to generate a historic plot of availability scores over time. This information may be analyzed by ML techniques to further influence future availability score calculations.

In 407, the wearable 200 determines whether the user is available for the inquiry 401, based on both the urgency score and the availability score. An operator may define a relationship between quantized urgency scores and quantized availability scores. Typically, a higher urgency score is needed to notify a user with a higher availability score. An operator may define any number of quantized urgency scores (e.g., five) and any number of availability scores (e.g., five). An operator may also define any relationship between urgency scores and availability scores.

An example is illustrative. If there are five urgency scores (1-5) and five availability scores (1-5) an operator may define the relationship between the availability and urgency scores such that the scores are added together, and a user is only notified of the inquiry 401 if the added score meets and/or exceeds a defined threshold. For example, if the threshold is 7, the urgency score is 3, and the availability score is four, the user would be notified (3+4=7). One of skill in the art will recognize that more complicated relationships between the threshold, availability score, and urgency score are possible. Additionally, the use of five quantized urgency and availability scores is only exemplary.

In 408, if the user is determined not to be available based on the availability score and urgency score in 407, the inquiry 401 may be placed into a de-prioritized location in a user worklist or inbox, and the user is not immediately notified.

If the user is determined to be available based on the availability score and urgency score in 407, a notification may inform the user of the inquiry 401. Alternatively, the inquiry 401 may be placed in a prioritized location in a user worklist or inbox, or a combination of notification and prioritization may occur.

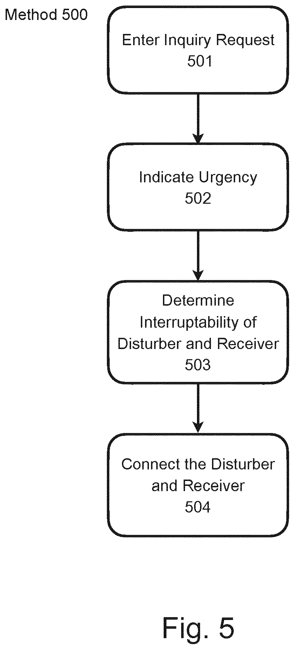

Fig. 5 shows a flow diagram 500 for interruption management based on urgency, according to various exemplary embodiments. As described above, interruption management may include manners for the 'disturber' (e.g., nurse, technician, colleague radiologist, etc.) to indicate how urgent the message is to the 'receiver' (e.g., radiologist). Throughout this description, the terms 'disturber' and `receiver' will be used. It should be understood that the 'disturber' is the person/entity that is sending an inquiry or request to the 'receiver' (e.g., the person that is going to be interrupted by the inquiry or request). Again, in the context of this description, it is described that the 'receiver' is a radiologist but that is only exemplary as the receiver may be any person that is going to be interrupted by an inquiry or request. In addition, throughout this description, the system implementing the interruption management may be termed an interruption management system. The interruption management system may be implemented for example, via the system 100 described above. However, the interruption management system is not limited to being implemented via the system 100 as it may be implemented on any system that includes the requisite hardware, software and/or firmware to operate in accordance with the operations described below. Furthermore, in some examples, where the disturber is requested to enter information regarding the inquiry or request, the information may be entered via a (mobile) application as described in greater detail below.

In 501, the disturber enters an inquiry that is directed toward a specific (or group of) radiologist(s). The inquiry may include a certain level of detail such as a quick description, title, and some case details. In some examples, depending on the context, using of systems to repopulate the relevant information (e.g., patient information, type of consult, urgency, etc.) is possible. For example, when a technologist uploads an acquisition to PACS it could be checked for potential findings, and if that is the case, automatically trigger a consult.

In some exemplary embodiments, the entering of the information by the disturber may be via a graphical user interface (GUI) that is presented by an application that may be executed by a device. For example, the GUI may be presented on the user interface 104 of system 100 or on the display device 215 of the wearable 200. This GUI may include fields (e.g., text boxes, drop down menus, etc.) that are fillable by the disturber. The GUI may also indicate the minimum amount of information that is used by the interruption management system to determine when to interrupt the receiver, e.g., asterisked fields that must be filled out to result in a complete request.

In 502, the disturber indicates how urgent the inquiry is in the form of a time interval in which the request is to be fulfilled (e.g., a question needs to be answered). Again, as described above, in some instances this urgency value may be automatically populated based on the type of request. In other examples, the disturber may manually indicate the urgency (e.g., via the exemplary GUI described above). In some examples, the GUI may include a drag bar such that the disturber can drag the interval time to the desired length. In further exemplary embodiments, the GUI may also display a suggested time interval based on an analysis by artificial intelligence (AI) or protocol information. When the urgency measure is set up, the inquiry is processed and prioritized accordingly. In the background, the continuous sensing of interruptability of the disturber and receiver may be initiated as will be described in greater detail below.

In 503, the interruption management system determines a time to connect the stakeholders (e.g., the disturber and the receiver). The interruption management system aligns interruptability curves of both the stakeholders. The interruptability curve may be based on a synthesized scoring system of multiple parameters such as focus/flow (e.g., by tracking eye-gaze activity and analyzing computer behaviour), stress levels (e.g., with Heart Rate Variability (HRV) sensing, or by integrating a system that measures stress, stress induced cognitive impairment can be estimated), case details (e.g., by analyzing complexity, urgency and type of case that is active indicated within a protocol), workflow phase (e.g., by recording the progress of the image review that is active), and location (e.g., by recording whether the stakeholders are behind their desk or walking through the building). Other parameters and other manners of tracking parameters related to a radiologist (or other persons) have been described above and these parameters and/or tracking methods may also be used in this exemplary embodiment.

To an example of a system that measures stress, Philips provides an Emography system that includes a wristband using galvanic skin response to measure and predict stress levels, and the resulting effect on cognitive performance, based on electro dermal response and cortisol level estimation algorithms. However, it should be understood that the exemplary embodiments are not limited to this type of stress measuring system, any system that measures stress level may be used by the exemplary embodiments.

Using these parameters, an estimated score is mapped onto a graph over time for both stakeholders. When both stakeholders are significantly interruptible around the same moment in time, a signal is sent to a communication bridge element. The operation of the communication bridge element will be described in greater detail below. In scenarios where the receiver may be one of multiple people (e.g., a group of radiologists), the interruptability of each of the members of the group may be determined on a continuous basis to find the most available person to contact. If the interruption management system determines that both stakeholders will not be available around the same period of time, then after the defined interval (e.g., the urgency time indicated by the disturber), the same signal is transferred to the communication bridge element.

The following provides examples of the parameters that may be used for the interruptability analysis. As described above, these parameters are only exemplary and other parameters may also be used. In a first example, the focus/flow score may range from -7 - 0 - 7 (just like all other parameters) and can be measured through combining eye-gaze behaviour, pupil dilation, body & head posture, system analysis and distraction sensing. This way it can be determined if the user is looking at the screen or looking away. Also, the diameter of the pupil may indicate how engaged the person is with what they are looking at. This combination creates a foundation to quantify focus. Complementary or alternatively attentiveness may be derived from measuring interactional state (key presses, mouse behaviour, selections, and states in the applications), or measuring contextual state (other people present, conversation analysis, etc.) where -7 is distracted and +7 is focussed.

In a second example, stress levels may range from -7 - 0 - 7 and can be measured through combining the heart-rate variability and skin conductance (or galvanic skin response (GSR)). Much research has been done on measuring stress, and these variables are the most commonly used and validated as most successful indicators of stress where -7 is calm and +7 is stressed.

In a third example, case complexity may range from -7 - 0 - 7 and can be measured through analyzing the data that is manually inputted when this case was created. Urgency, complexity, and type of case can be derived and processed to one output of case complexity, wherein -7 is simple and +7 is complex.

In a fourth example, workflow phase may range from -7 - 0 - 7 and can be measured through analysing system activity and eye-gaze behavior. Examples of separate phases in the workflow are preparing, exploring, reviewing, analyzing, reporting, and concluding. The process of, for example, an image review can be analyzed by how many steps within the phases have been taken and what portion of the image has already been reviewed wherein -7 is the beginning and +7 is the end.

The workflow phase may also include information about the type of workflow, e.g., clinical exams vs. outpatient exams. Depending on the clinical problem it can be predicted how urgent the reading is needed. For example, pulmonary embolism within 1 hour, aorta aneurysm/bleeding within 10 minutes, stroke within 1 minute, etc. By linking this system to the worklist of modalities and the clinical request information a prediction of when a communication is anticipated can be made. These types of workflows can also be scored using the scale described herein.

In a fifth example, location may range from -7 - 0 - 7 and can be measured through GPS data sources (or beacons) that are derived by the phone of the radiologist, or an alternative device (e.g., RFID card) that the radiologist carries. This way it can be determined when the radiologist is walking and more available for a phone call where -7 indicates the radiologist is at their desk and +7 indicates the radiologist is walking.

These different data sources are then synthesized into one interruptability score. In one example, this may be done via a formula that describes the relations between the measurables as follows: Predicted Interruptability score = (focus/flow * - significance) + (stress * - significance) + (case complexity * - significance) + (workflow * significance) + (location * significance).

The significance scores may be initially defined by an assumed distribution based on an importance of the parameter. Therefore, these scores are different for every parameter and are optimized and iterated over time based on the feedback that stakeholders can give after being interrupted. Providing feedback is an optional functionality. Some significance scores are negative and some are positive meaning that e.g., high focus, stress, or case complexity scores result in a lower interruptability score. It should be understood that the above formula is only exemplary and other manners of combining the parameters to result in an interruptability score may also be used.

When these interruptability scores are analyzed, estimated, and plotted on a curve, optimized moments of interruption can be determined. An estimation of the coming availability levels can be approached via analyzing the work schedule of the individual stakeholders. The simplest way would be to look at when the next "gap" is expected or when a radiologist is approximately done with reporting a case. Additionally, in some exemplary embodiments, stress induced cognitive impairment can be measured and analyzed via technologies and algorithms used in, for example, the Philips Emography system described above. This way, over time an increasingly accurate prediction can be made of upcoming availability.

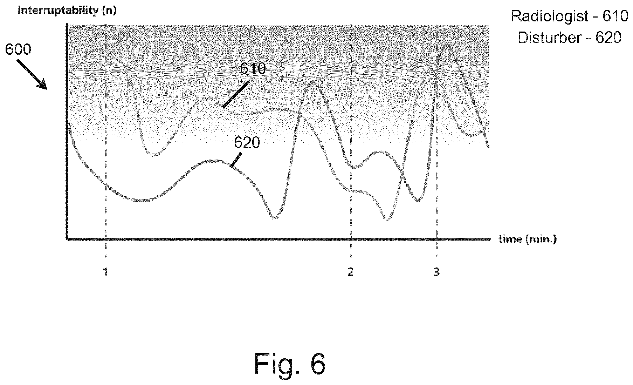

When the interruptability curves of both stakeholders are overlayed onto one another, more insight is given on when the optimal moment is to interrupt. Fig. 6 shows an exemplary graph 600 plotting the interruptability curves of two stakeholders according to various exemplary embodiments. In the example of Fig. 6, two interruptability curves are shown, an interruptability curve 610 for the radiologist and an interruptability curve 620 for the disturber. The interruptability curves are plotted along an x-axis that is the time interval for which the disturber requested the inquiry to be completed and the y-axis which is a measure of the interruptability score.

Fig. 6 shows a visual representation that is the foundation of finding the optimal moment to disturb the involved stakeholders. When the curve is entering the gradient area, people should be (to a certain extent) acceptably interruptible. Three key moments are highlighted to give more insight into how this interruption management system operates.

At moment 1, the disturber (interruptability curve 620) is very available, while the radiologist (interruptability curve 610) is not. Therefore, this is not the right moment to interrupt. If this situation would be the other way around (e.g., the radiologist very available while the disturber is not), the interruption management system may consider this to be an optimal moment for the interruption because the radiologist is generally under more pressure and prioritized as opposed to the disturber. In that scenario, it is recommended to align the inquiry subject with the activity that the disturber is doing. If it is an activity that has less priority than the inquiry, the interruption is allowed to occur.

At moment 2, both stakeholders are not available so the interruption management system would not allow the interruption to occur.

At moment 3, both stakeholders are available so the interruption management system would provide a signal to the communication bridge element to setup a call between the involved stakeholders.

In 504, the communication bridge element connects the disturber and receiver via a communication bridge. For example, the communication bridge element may automatically set up a call between the disturber and the receiver based on the signal received by the communication bridge element as described above. As also described above, in some instances the receiver may be one of a set of persons (e.g., one of a set of radiologists currently on duty). The signal may include an identification of both the disturber and the correct one of the receivers so that the communication bridge element initiates the call between the correct stakeholders. Thus, when the signal is received, the communication bridge element calls both stakeholders and facilitates the channel of communication. The "call" may be performed via phone or mobile phone, but it can also be facilitated through other platforms such as MS Teams, Skype, Zoom, WebEx, or whatever communication service is used in that hospital. In other examples, the "call" can be text messages and/or a chat function.

As described above, in some exemplary embodiments, feedback may be received from both stakeholders on how interruptible both stakeholders actually were when they were interrupted (e.g., based on self-reporting). This feedback may be used to adjust the specific significance scores. The amount of change in this variable can then be translated towards a correction in threshold and weighting.

In some exemplary embodiments, the interruption management system detects when a radiologist or technologist is on the phone. When this is detected, the availability score is immediately set to the minimum. This way no disturbing phone calls can be running in parallel. This can be automatically sensed via activity on the phone itself.

In some exemplary embodiments, the interruption management system may have insights into the flow of both stakeholders and this information may be shared among the stakeholders. However, in some exemplary embodiments this feature may be deactivated by default because it is expected to be distracting.

The methods described in relation to Figs. 3-5, and the system described in relation to Figs. 1-2, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram in Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Those skilled in the art will understand that the above-described exemplary embodiments may be implemented in any suitable software or hardware configuration or combination thereof. An exemplary hardware platform for implementing the exemplary embodiments may include, for example, an Intel x86 based platform with compatible operating system, a Windows OS, a Mac platform and MAC OS, a mobile device having an operating system such as iOS, Android, etc. In a further example, the exemplary embodiments of the above-described method may be embodied as a program containing lines of code stored on a non-transitory computer readable storage medium that, when compiled, may be executed by a processor or microprocessor.

Although this application described various aspects each having different features in various combinations, those skilled in the art will understand that any of the features of one aspect may be combined with the features of the other aspects in any manner not specifically disclaimed or which is not functionally or logically inconsistent with the operation of the device or the stated functions of the disclosed aspects.

It is well understood that the use of personally identifiable information should follow privacy policies and practices that are generally recognized as meeting or exceeding industry or governmental requirements for maintaining the privacy of users. In particular, personally identifiable information data should be managed and handled so as to minimize risks of unintentional or unauthorized access or use, and the nature of authorized use should be clearly indicated to users.

It will be apparent to those skilled in the art that various modifications may be made in the present disclosure, without departing from the spirit or the scope of the disclosure. Thus, it is intended that the present disclosure cover modifications and variations of this disclosure provided they come within the scope of the appended claims and their equivalent.

## Claims

1. A method, comprising:
generating attention data of a user, wherein the attention data comprises at least user sensor data and a current case status, wherein the current case status comprises one of a case complexity, a case urgency, or a progression of image review;
determining the occurrence of an interruption or a change in the attention data; and
performing an adjustment of one or more workspace items based on the determined occurrence.

2. The method of claim 1, wherein the user sensor data comprises one of user eye-gaze behavior, user posture, user heart-rate variability, user skin conductance, user pupil dilation, or detected distractions nearby a user.

3. The method of claim 1, wherein the adjustment is based on a focus preference.

4. The method of claim 1, wherein performance of the adjustment further comprises adjusting at least one of a noise cancellation system, a lighting control system, an ergonomics system, a scent system, or a sound system.

5. The method of claim 1, wherein determining the occurrence of an interruption further comprises a user state, the user state comprising at least one of attentiveness, fatigue, or stress.

6. The method of claim 5, wherein the user state is a quantized value corresponding to a predefined metric.

7. The method of claim 6, wherein the performance of the adjustment is based on the quantized value.

8. A computer program comprising computer program code means adapted, when said computer program is run on a computer, to implement the method of any of claims 1-7.

9. A system, comprising
one or more processors and
at least one memory with instructions that, when executed by the one or more processors, cause the one or more processors to:
generate attention data of a user, wherein the attention data comprises user sensor data and current case status;
determine the occurrence of an interruption or a change in the attention data; and
perform or adjust one or more workspace items based on the determined occurrence.

10. The system of claim 9, wherein the user sensor data comprises one of user eye-gaze behavior, user posture, user heart-rate variability, user skin conductance, user pupil dilation, or detected distractions nearby a user.

11. The system of claim 9, wherein the adjustment is based on a focus preference.

12. The system of claim 9, wherein the adjustment further comprises adjusting at least one of a noise cancellation system, a lighting control system, an ergonomics system, a scent system, or a sound system.

13. The system of claim 9, wherein the occurrence of an interruption further comprises a user state, the user state comprising at least one of attentiveness, fatigue, or stress.

14. The system of claim 13, wherein the user state is a quantized value corresponding to a predefined metric.

15. The system of claim 14, wherein the performance of the adjustment is based on the quantized value.
